(19) Europäisches Patentamt
European Patent Office
Office européen des brevets

(11) Veröffentlichungsnummer: **0 127 000**
**B1**

(12) # EUROPÄISCHE PATENTSCHRIFT

(45) Veröffentlichungstag der Patentschrift: **07.01.87**

(51) Int. Cl.⁴: **C 07 C 93/187**, A 61 K 31/16

(21) Anmeldenummer: **84104880.4**

(22) Anmeldetag: **02.05.84**

(54) **1,3-bis(Dimethylamino)-2-propyl-4-chlorphenoxyacetat, seine Säureadditionssalze, Verfahren zu seiner Herstellung und diese Verbindungen enthaltende Arzneimittelpräparate.**

(30) Priorität: **02.05.83 HU 149683**

(43) Veröffentlichungstag der Anmeldung: **05.12.84 Patentblatt 84/49**

(45) Bekanntmachung des Hinweises auf die Patenterteilung: **07.01.87 Patentblatt 87/2**

(84) Benannte Vertragsstaaten: **AT BE CH DE FR GB IT LI NL SE**

(56) Entgegenhaltungen: **Chemical Abstracts Band 84, Nr. 3, 19. January 1976, Columbus, Ohio, USA; Seite 430, Spalte 2, Abstract Nr. 16978v**

(73) Patentinhaber: **BIOGAL GYOGYSZERGYAR, Pallagi u. 13., H-4042 Debrecen (HU)**

(72) Erfinder: **Nagy, Imre Zs., Dr., Nagyerdei krt. 2, H-4042 Debrecen (HU)**
Erfinder: **Emri geb. Hársy, Zsuzsanna, Dr., Kartács u. 36, H-4042 Debrecen (HU)**
Erfinder: **Jancso, Sándor, Dr., Kardos u. 28, H-4042 Debrecen (HU)**
Erfinder: **Csernus, István, Dipl.-Chem., Batthány 17-19, H-4042 Debrecen (HU)**
Erfinder: **Bálint, János, Dr., Péchy u. 5, H-4042 Debrecen (HU)**

(74) Vertreter: **Hansen, Bernd, Dr.rer.nat. et al, Hoffmann, Eitle & Partner Patentanwälte Arabellastrasse 4, D-8000 München 81 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 127 000 B1

ACTORUM AG

**Beschreibung**

Die Erfindung betrifft die neue Verbindung 1,3-bis-(Dimethylamino)-2-propyl-4-chlorphenoxyacetat (Formel I)

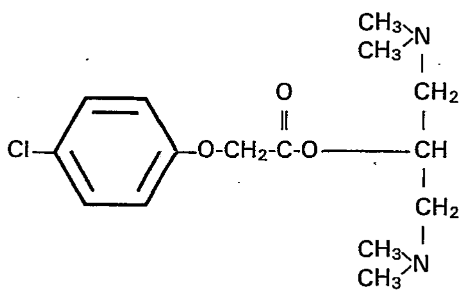

(I)

ihre Säureadditionssalze, ein Verfahren zur Herstellung der Verbindung sowie Arzneimittelpräparate, die die Verbindung beziehungsweise ihre Säureadditionssalze enthalten. Die Verbindung der Formel (I) wirkt als Neurostimulant und ist fähig, altersbedingte Funktionsausfälle des Gehirns rückgängig zu machen.

Es ist bekannt, dass 4-Chlorphenoxy-essigsäure-β-dimethylaminoäthylester eine das Erinnerungsvermögen stimulierende, neuroenergetische Wirkung hat [Martin Regwer: Organisch-chemische Arzneimittel und ihre Synonyma, Akademie-Verlag Berlin 1951 (1978)]. Das diese Verbindung enthaltende Arzneimittel (Centrophenoxin) hat als zentrales Stimulans Eingang in die ärztliche Praxis gefunden [Arzneimittelforschung 13, 881 (1963)]. Zur Herstellung der Verbindung wurden zahlreiche Verfahren ausgearbeitet. Bei der überwiegenden Mehrzahl handelt es sich um übliche Veresterungsverfahren [tschechoslowakische Patentschrift Nr. 154 866, japanische Patentanmeldung (Kokai) Nr. 72-389387, britische Patentschrift Nr. 954 196, DT-OS Nr. 2 006 338], gemäss der japanischen Patentanmeldung (Kokai) Nr. 75-19542 wird der erhaltene Ester nachträglich chloriert, und gemäss der japanischen Patentanmeldung Nr. (Kokai) 75-19542 kann die Verbindung auch durch eine Dreikomponentenreaktion (4-Chlorphenol, Chloressigsäure und 1,3-bis(dimethylamino)-2-propanol) erhalten werden.

Bei der Untersuchung des Wirkungsmechanismus der genannten Verbindung stellt sich heraus, dass die ursprünglich nur als Neuroenergetikum bekannte Substanz auch auf die Alterungsprozesse der Zellen einwirkt. So wurde festgestellt, dass bei alten Tieren durch die Wirkung einer chronischen Behandlung der Gehalt an Alterungspigment in den Nervenzellen des Gehirns und im Myocard geringer wurde [Nature 210, 313 (1966)], das Lebensalter der Tiere verlängert wurde und die Lernfähigkeit der alten Tiere sich besserte [Expl. Gerontol. 8, 185 (1973b)].

Gemäss der Membranhypothese der Zellalterung besteht der erste Schritt des Alterns der postmitotischen Zelle darin, dass die passive Kaliumpermeabilität der Zellmembran geringer wird, was sicher auf die Querbindungsfähigkeit der bei der Zellatmung entstehenden aggressiven freien Radikale zurückzuführen ist [J. Theor. Biol. 75, 189 (1978); Mech. Age. Dev. 9, 237 (1979)]. Die oben genannte Verbindung hat an ihrer substituierten Aminogruppe in vitro eine beträchtliche radikalneutralisierende Wirkung, die wahrscheinlich mit der Fähigkeit des Stickstoffatoms zur Elektronenabgabe in Zusammenhang steht [Mech. Age. Dev. 14, 245 (1980)].

Die aus der Literatur bekannten stickstoffhaltigen 4-Chlor-phenoxy-essigsäureester — so auch das Centrophenoxin — enthalten eine substituierte Aminogruppe [Bull. Soc. Chim. France 1960, 1786; Biol. Aktiv. Soedin 1965, 112; C.A., 63, 17948c (1965)]. Die Erfindung geht nun von der Annahme aus, dass der Dimethylaminoäthanolteil des Moleküls des bekannten Wirkstoffes in das Gehirngewebe eingebaut wird und dort die freien Radikale neutralisiert. Wenn dem so ist, muss weiter angenommen werden, dass dabei zwei Aminogruppen wirksamer sind als eine und durch Synthese einer Analogverbindung mit zwei substituierten Aminogruppen ein wirksameres Mittel gegen die Alterungsprozesse der Zellen hergestellt werden kann.

Gegenstand der Erfindung sind demnach die Verbindung 1,3-bis(Dimethylamino)-2-propyl-4-chlorphenoxyacetate und ihre Säureadditionssalze. Gegenstand der Erfindung ist weiter ein Verfahren zur Herstellung der Verbindung. Erfindungsgemäss wird die Verbindung der Formel (I) hergestellt, indem man eine Verbindung der allgemeinen Formel (II)

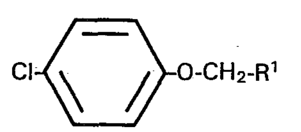

(II)

worin

$R^1$ für Carboxyl-, Cyano-, Alkokycarbonyl-, Halogencarbonyl-, 4-Chlorphenoxy-acetyloxycarbonylgruppe oder eine Gruppe der Formel -COOMe$^I$ mit Me$^I$ = Alkalimetall- oder Ammonium steht,

mit einer Verbindung der allgemeinen Formel (III)

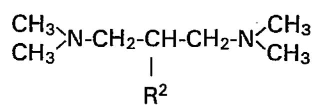

(III)

worin

$R^2$ für Hydroxylgruppe oder Halogenatom steht, umsetzt und die gebildete Zielverbindung — vorzugsweise in Form eines Säureadditionssalzes — aus dem Reaktionsgemisch isoliert.

Die Esterbildung kann gemäss den üblichen und literaturbekannten Verfahren vorgenommen werden (S. Patai: The chemistry of carboxylic acids and esters, Wiley, London 1969). Zum Beispiel kann die freie Säure ($R^1$ = Carboxyl) mit dem Alkohol oder dessen Halogenderivat ($R^2$ = CH bzw. Hal) in einem Lösungsmittelmedium umgesetzt werden. Statt der freien Säure können auch ihre funktionellen Derivate wie das Nitril ($R^1$ = CN), das Anhydrid ($R^1$ = 4-Chlorphenoxyacetylcarbonyl), das Halogenid ($R^1$ = Halogencarbonyl) oder ein Ester ($R^1$ = Alkoxycarbonyl) eingesetzt werden.

Vorzugsweise verwendet man als Säurekomponente 4-Chlorphenoxyessigsäurechlorid (oder -nitril) und setzt dieses in einem organischen Lösungsmittelmedium, zum Beispiel Benzol oder Xylol, bei erhöhter Temperatur mit dem 1,3-bis(Dimethylamino)--2-propanol um. Die Reaktion ist bei 60-80°C Reaktionstemperatur nach 2-3 Stunden beendet. Das Reaktionsprodukt wird vorzugsweise in Form eines Hydrochlorids aus dem Reaktionsgemisch isoliert.

Die akute Toxizität des neuen Wirkstoffes wurde an männlichen CFLP-Albinoratten intraperitoneal und oral untersucht. Die nach Lichtfield-Wilcoxon ermittelten $LD_{50}$-Werte sind folgende: intraperitoneal $LD_{50} = 700 \pm 42$ mg/kg, per os $LD_{50} = 2540 \pm 300$ mg/kg.

Die Wirkung der Verbindung der Formel (I) wurde auch hinsichtlich der Überlebensdauer untersucht. Fünf CFY-Ratten erhielten i.p. täglich 100 mg/kg Wirkstoff, gelöst in physiologischer Kochsalzlösung. Das Durchschnittslebensalter dieser Ratten war um etwa 5 Monate länger als das der Tiere in der Kontrollgruppe.

Ferner wurde die Wirkung der Verbindung auf biologische Membranen, insbesondere die Zellmembranen der Gehirnrinde, untersucht. Es wurde gefunden, dass die Mikroviskosität der Membranen der aus der Gehirnrinde isolierbaren Sinaptosomen mit ansteigendem Lebensalter beträchtlich wächst. (Die Mikroviskosität wurde durch Messung der Fluoreszenz-Anisotropie der mit Diphenylhexatrien markierten Membranen bestimmt.) 24 Monate alte CFY-Ratten wurden 20 Tage lang mit täglich 100 mg/kg i.p. des Wirkstoffes behandelt, und durch die Behandlung besserte sich der genannte Parameter um so viel, dass er etwa mit dem im Alter von einem Jahr messbaren Parameter identisch war. Dieses Ergebnis steht in völliger Übereinstimmung mit der durchschnittlichen Verlängerung des Lebensalters.

Die erfindungsgemässe Verbindung wirkt auch auf die RNS-Synthese der Gehirnzellen. Die Total- und die mRNS-Synthese der Gehirnrindenzellen alter Ratten (24 Monate alt) geht etwa um die Hälfte langsamer vor sich als im Falle junger und erwachsener Tiere. Eine über 4 Wochen hinweg mit täglich 100 mg/kg i.p. des Wirkstoffes vorgenommene Behandlung der alten Tiere hatte das Ergebnis, dass die Synthese beider RNS-Fraktionen bedeutend beschleunigt wurde und beinahe wieder den an einjährigen Tieren messbaren Wert erreichte.

Aufgrund dieser Ergebnisse ist zu erwarten, dass der neue Wirkstoff die Funktion der Gehirnzellen und damit die geistige Tätigkeit (einschliesslich der Assoziations- und der Lernfähigkeit) stimuliert, insbesondere in Fällen, in denen diese Fähigkeiten entweder durch den natürlichen Alterungsprozess oder durch organische Krankheiten beziehungsweise traumatische Zustände vermindert wird. Die Verbindung ist daher als Neurostimulans sowie als prophylaktisches Mittel gegen die Herausbildung von Funktionsstörungen des Gehirns infolge Alterns (als «antiaging» Mittel) geeignet.

Die Erfindung betrifft auch Arzneimittelzubereitungen, die die neue Verbindung oder deren Säureadditionssalze enthalten. Die Arzneimittelzubereitungen können zur Einnahme per os geeignete Tabletten sein, die den Wirkstoff zusammen mit den in der Arzneimittelindustrie üblichen Träger- und Hilfsstoffen (Stärke, Cellulose, Talkum) enthalten. Zur Einnahme per os kann der Wirkstoff auch als Kapsel oder Dragee formuliert werden. Zur Applikation i.v. wird der Wirkstoff in Wasser, physiologischer Kochsalzlösung oder einem physiologisch unbedenklichen organischen Lösungsmittel (unterschiedliche Glycole) gelöst. Zur Anwendung intramuscular wird der Wirkstoff in der gleichen Weise gelöst oder aber suspendiert. Die festen Formulierungen enthalten im allgemeinen 20-90% Wirkstoff, die Lösungen 1 bis 10% und die Suspensionen 1-70%.

Die Erfindung wird im folgenden anhand von Beispielen näher erläutert.

*Beispiel 1*

13,0 g (0,063 Mol) 4-Chlorphenoxyessigsäurechlorid werden in 140 ml Benzol gelöst. Die Lösung wird auf 0°C gekühlt und unter Rühren tropfenweise mit 18,2 g (0,126 Mol) 1,3-bis(Dimethylamino)-2-propanol versetzt, wobei darauf zu achten ist, dass die Temperatur nicht über 10°C ansteigt. Das Reaktionsgemisch wird am Rückfluss 3 Stunden lang gekocht. Dann wird das Lösungsmittel im Vakuum abdestilliert. Das als Rückstand verbleibende 1,3-bis-(Dimethylamino)-2-propyl-4-chlorphenoxyacetat wird in einem im Verhältnis 10:1 bereiteten Gemisch aus Diäthyläther und Aceton gelöst und durch Einleiten von Salzsäuregas in Form seines Hydrochlorids ausgefällt. Das Rohprodukt wird abfiltriert, mit Äther gewaschen und aus Isopropanol umkristallisiert. Man erhält 21,0 g (85,5%) Produkt, das bei 210 bis 212°C schmilzt.

Analyse für $C_{15}H_{23}ClN_2O_3 \cdot 2HCl$ (M = 387,7)

| | | | | |
|---|---|---|---|---|
| ber.: | C 46,46 | H 6,50 | N 7,22 | Cl 27,43% |
| gef.: | C 45,42 | H 6,56 | N 7,70 | Cl 28,26% |

$\lambda_{max}$ (in Methanol): 276 nm, log ε: 1,5104

IR (KBr): 2580 (Aminsalz), 1765 (Estercarbonyl), 1215, 1080 (Arylalkyläther), 820 (aromatische Gruppe in p-Stellung) $cm^{-1}$

$^1$H-NMR (DMSO): δ 7,23-7,17 (m, aromatisch), 5,71 (s, CH), 5,20 (s, O-$CH_2$), 3,47 (s, $CH_2$), 2,90 (s, $CH_3$).

*Beispiel 2*

6,5 g (0,032 Mol) 4-Chlorphenoxyessigsäurechlorid werden in 120 ml Toluol gelöst. Die Lösung wird auf 0°C gekühlt und bei einer Temperatur unter 10°C tropfenweise mit 9,4 g (0,064 Mol) 1,3-bis-(Dimethylamino)-2-propanol versetzt. Das Reaktionsgemisch wird drei Stunden lang auf 80-82°C erwärmt, dann auf Raumtemperatur gekühlt und das 1,3-bis(Dimethylamino)-2-propyl-4-chlorphenoxyacetat durch Einleiten von Salzsäuregas als Dihydrochlorid ausgefällt. Das Rohprodukt wird abfiltriert, mit Isopropanol gewaschen und aus Isopropanol umkristallisiert. Man erhält 10,1 g (82,2%) Produkt, das bei 209-211°C schmilzt. Die übrigen physikalischen Konstanten stimmen mit denen des Produktes gemäss Beispiel 1 überein.

*Beispiel 3*

6,0 g (0,017 Mol) (4-Chlorphenoxy)-essigsäureanhydrid werden in 70 ml Benzol gelöst. Die Lösung wird bei 5-10°C unter Rühren mit 5,6 g (0,019 Mol) 1,3-bis(Dimethylamino)-2-propanol versetzt. Das

Reaktionsgemisch wird drei Stunden lang gekocht und dann das Lösungsmittel im Vakuum abdestilliert. Der Rückstand wird in einem im Verhältnis 10:1 bereiteten Gemisch von Diäthyläther und Aceton gelöst und dann durch Einleiten von Salzsäuregas das Hydrochlorid ausgefällt. Das Produkt wird abfiltriert und aus Isopropanol umkristallisiert. Man erhält 5,7 g (87,02%) Produkt, das bei 209-211°C schmilzt und in allen Parametern mit dem Produkt gemäss Beispiel 1 übereinstimmt.

*Beispiel 4*

15,0 g (0,085 Mol) (4-Chlorphenoxy)-essigsäurenitril werden in 80 ml Toluol gelöst. Bei 5-10°C werden zu der Lösung 14,7 g (0,1 Mol) 1,3-bis(Dimethylamino)-2-propanol und 2,0 ml 85%ige Schwefelsäure gegeben. Das Reaktionsgemisch wird am Rückfluss 5 Stunden lang gekocht, dann auf Raumtemperatur abgekühlt und mit Natriumcarbonatlösung neutralisiert. Die neutrale organische Phase wird mit Wasser gewaschen, über Natriumsulfat getrocknet und dann mit Salzsäure gesättigt. Das ausgefallene 1,3-bis(Dimethylamino)-2-propyl-4-chlorphenoxyacetat-dihydrochlorid wird abfiltriert, getrocknet und aus Isopropanol umkristallisiert. Man erhält 25,67 g (78,2%) Hydrochlorid, das bei 208-210°C schmilzt und in seinen sonstigen Parametern mit dem Produkt gemäss Beispiel 1 identisch ist.

*Beispiel 5*

18,66 g (0,1 Mol) (4-Chlorphenoxy)-essigsäure werden in 40 ml Isopropanol gelöst. Zu der Lösung gibt man unter Rühren tropfenweise die mit Isopropanol bereitete Lösung von 16,70 g (0,1 Mol) 1,3--bis(Dimethylamino)-2-chlorpropan. Das Reaktionsgemisch wird 2 Stunden lang gekocht, dann abgekühlt, mit 200 ml Diäthyläther verdünnt und mit Salzsäregas gesättigt. Das Hydrochlorid wird abfiltriert und mit Äther gewaschen. Man erhält 31,2 g (80,5%) Produkt, das bei 208-210°C schmilzt und in seinen sonstigen Parametern mit dem Produkt gemäss Beispiel 1 übereinstimmt.

*Beispiel 6*

Man arbeitet ähnlich wie gemäss Beispiel 5, verwendet jedoch als Säurekomponente 20,86 g (0,1 Mol) (4-Chlorphenoxy)-essigsäure-natriumsalz, gelöst in 40 ml Dimethylformamid. Das erhaltene Dihydrochlorid (30,02 g = 85%) stimmt in seinen Eigenschaften mit dem Produkt gemäss Beispiel 1 überein.

*Beispiel 7*

Ein Gemisch aus 10,0 g (0,05 Mol) (4-Chlorphenoxy)-essigsäuremethylester, 7,35 g (0,05 Mol) 1,3--bis(Dimethylamino)-2-propanol, 1,9 g Natriumäthylat und 30 ml Benzol wird 10 Stunden lang gekocht, wobei der entstehende Methylalkohol in Form seines mit Benzol gebildeten azeotropen Gemisches kontinuierlich aus dem Reaktionsgemisch abdestilliert wird. Die zurückbleibende Lösung wird im Vakuum eingedampft. Der Rückstand wird in einem im Verhältnis 10:1 bereiteten Gemisch aus Diäthyläther und Aceton gelöst und durch Einleiten von Salzsäuregas in Form seines Hydrochlorids ausgefällt. Das Produkt wird abfiltriert und aus Isopropanol umkristallisiert. Man erhält 14,80 g (76,5%) Produkt, das bei 207-210°C schmilzt und in seinen sonstigen Parametern mit dem Produkt gemäss Beispiel 1 übereinstimmt.

*Beispiel 8*

Tabletten mit 250 mg Wirkstoffgehalt

| | |
|---|---|
| Wirkstoff | 250 mg |
| Lactose | 45 mg |
| kristalline Cellulose | 17 mg |
| Talkum | 5 mg |
| Paraffinöl | 8 mg |

2500 g kristallines 1,3-bis(Dimethylamino)-2-propyl-4-chlorphenoxyacetat, 450 g wasserfreie Lactose, 170 g wasserfreie kristalline Cellulose werden in einem Mischer mit 80 g Paraffinöl und 350 ml wasserfreiem Isopropanol homogenisiert. Die Masse wird durch ein Sieb gedrückt und zu Granulen getrocknet. Die getrocknete Masse wird mit 50 g Talkum vermischt und dann in der üblichen Weise auf der Tablettiermaschine zu Tabletten des Gewichtes von 325 mg verpresst.

*Beispiel 9*

Enterosolvente Kapseln oder Dragees

Das gemäss Beispiel 8 hergestellte Produkt wird auf an sich bekannte Weise mit einem enterosolventen Überzug versehen.

*Beispiel 10*

Kapseln

Jede Kapsel enthält

| | |
|---|---|
| Wirkstoff | 250 mg |
| bekannte Hilfsstoffe | qu. s. |

Der Wirkstoff wird mit den Hilfsstoffen homogenisiert und dann in Hartgelatinekapseln gefüllt.

*Beispiel 11*

Pulverampullen mit 250 bzw. 500 mg Wirkstoffgehalt

Der Wirkstoff wird in einer Menge von je 250 mg bzw. 500 mg in Pulverampullen gefüllt. Zur Anwendung wird die Ampulle mit 10 ml dest. Wasser aufgefüllt.

**Patentansprüche**

1. 1,3-bis(Dimethylamino)-2-propyl-4-chlorphenoxyacetat der Formel (I)

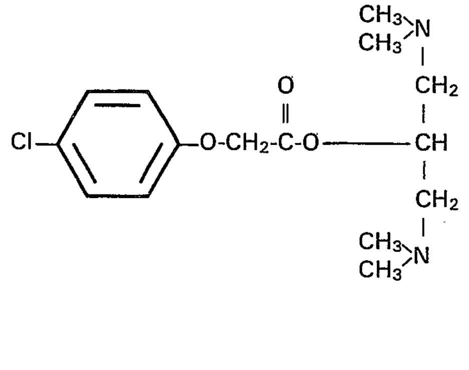

(I)

und seine physiologisch verträglichen Säureadditionssalze.

2. 1,3-bis(Dimethylamino)-2-propyl-4-chlorphenoxyacetat-dihydrochlorid.

3. Verfahren zur Herstellung von 1,3-bis(Dimethylamino)-2-propyl-4-chlorphenoxyacetat und seiner Säureadditionssalze, dadurch gekennzeichnet, dass man Verbindungen der allgemeinen Formel (II)

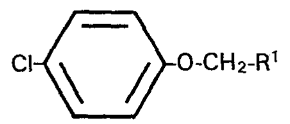 (II)

worin

$R^1$ für Carboxyl-, Cyano-, Alkokycarbonyl-, Halogencarbonyl-, 4-Chlorphenoxy-acetyloxycarbonylgruppe oder eine Gruppe der Formel -COOMe' mit Me' = Alkalimetall- oder Ammonium steht,

mit einer Verbindung der allgemeinen Formel (III)

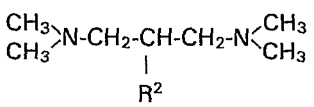 (III)

worin

$R^2$ für Hydroxylgruppe oder Halogenatom steht, umsetzt und die gebildete Zielverbindung — gegebenenfalls in Form eines Säureadditionssalzes — aus dem Reaktionsgemisch isoliert.

4. Verfahren nach Anspruch 3, dadurch gekennzeichnet, dass man als Ausgangsstoffe (4-Chlorphenoxy)-essigsäurechlorid und 1,3-bis(Dimethylamino)-2-propanol verwendet.

5. Arzneimittelpräparate, gekennzeichnet durch einen Gehalt an 1,3-bis(Dimethylamino)-2-propyl-4-chlorphenoxyacetat oder seinen Säureadditionssalzen.

6. Verwendung von 1,3-bis(Dimethylamino)-2-propyl-4-chlorphenoxyacetat oder von dessen Säureadditionssalzen für die Herstellung eines Arzneimittels zur Stimulierung der Gehirnfunktionen.

**Claims**

1. 1,3-bis(dimethylamino)-2-propyl-4-chlorophenoxyacetate of formula (I)

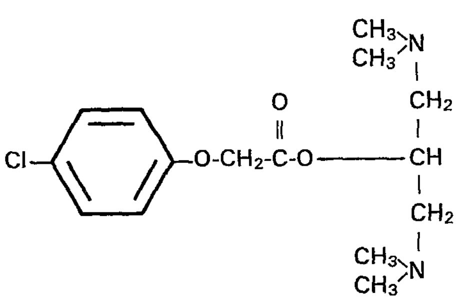 (I)

and its physiologically acceptable acid addition salts.

2. 1,3-bis(dimethylamino)-2-propyl-chlorophenoxyacetate-dihydrochloride.

3. Process for the production of 1,3-bis(dimethylamino)-2-propyl-4-chlorophenoxyacetate and its acid addition salts, characterized in that compounds of general formula (II)

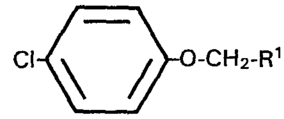 (II)

in which

$R^1$ is a carboxyl, cyano, alkoxycarbonyl, halogenocarbonyl, or a 4-chlorophenoxy-acetyloxycarbonyl group or a group of the formula -COOMe' where Me' = alkali metal or ammonium ion,

is reacted with a compound of the general formula (III)

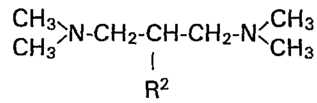 (III)

in which

$R^2$ is a hydroxyl group or a halogen atom and the target compound formed — if necessary in the form of its acid addition salt — is isolated from the reaction mixture.

4. Process according to claim 3 characterized in that (4-chlorophenoxy)-acetyl chloride and 1,3-bis-(dimethylamino)-2-propanol are used as starting materials.

5. Drug preparation, characterized in that it contains 1,3-bis(dimethylamino)-2-propyl-4-chlorophenoxyacetate or its acid addition salts.

6. Use of 1,3-bis(dimethylamino)-2-propyl-4-chlorophenoxyacetate or its acid addition salts for the preparation of a medicament for the stimulation of brain function.

**Revendications**

1. Le 4-chlorophénoxyacétate de 1,3-bis-(diméthylamino)-2-propyle de formule I

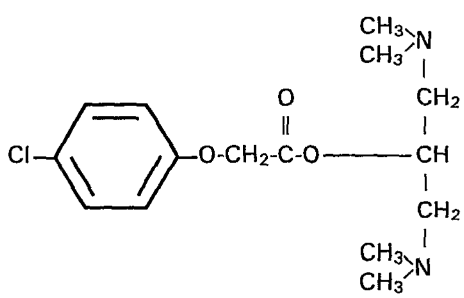 (I)

et ses sels formés par addition avec des acides acceptables pour l'usage pharmaceutique.

2. Le dichlorhydrate du 4-chlorophénoxyacétate de 1,3-bis-(diméthylamino)-2-propyle.

3. Procédé de préparation du 4-chlorophénoxyacétate de 1,3-bis-(diméthylamino)-2-propyle et de ses sels formés par addition avec des acides, caractérisé en ce que l'on fait réagir des composés de formule générale II

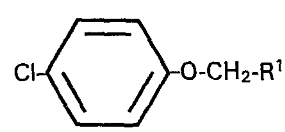 (II)

dans laquelle

$R^1$ représente un groupe carboxyle, cyano, alcoxycarbonyle, halogénocarbonyle, 4-chlorophénoxy-acétyloxycarbonyle ou un groupe de formule -COOMe$^I$ dans lequel Me$^I$ représente un ion de metal alcalin ou un ion ammonium, avec un composé de formule générale III

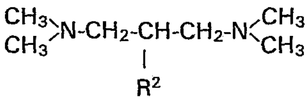

(III)

dans laquelle

$R^2$ représente un groupe hydroxy ou un atome d'halogène, et on isole le composé recherché — éventuellement à l'état de sel d'acide — du mélange de réaction.

4. Procédé selon la revendication 3, caractérisé en ce que l'on utilise en tant que produits de départ le chlorure de l'acide 4-chlorophénoxyacétique et le 1,3-bis-(diméthylamino)-2-propanol.

5. Préparations médicamenteuses caractérisées en ce qu'elles contiennent du 4-chlorophénoxyacétate de 1,3-bis-(diméthylamino)-2-propyle ou ses sels formés par addition avec des acides.

6. Utilisation du 4-chlorophénoxyacétate de 1,3-bis-(diméthylamino)-2-propyle ou de ses sels formés par addition avec des acides dans la préparation d'un médicament servant à la stimulation des fonctions cérébrales.